# EUROPEAN PATENT APPLICATION

(11) **EP 4 703 373 A1**
(43) Date of publication of application: **04.03.2026**
(21) Application number: 23934389.0
(22) Date of filing: 24.04.2023
(51) Int. Cl.: C07K 14/575, C07K 7/00, A61K 38/22, A61K 8/64, A61Q 7/00, A61P 17/10, A61P 17/14

(54) **POLYPEPTIDES AND RELATED SUBSTANCES AGAINST HAIR LOSS AND DERMATITIS, AND USES THEREOF**

(71) Applicant: Shenzhen Institutes of Advanced Technology, Shenzhen, Guangdong 518055 (CN)
(72) Inventor: ZHANG, Jian, Shenzhen, Guangdong 518055 (CN); YANG, Yali, Shenzhen, Guangdong 518055 (CN); GE, Lei, Shenzhen, Guangdong 518055 (CN); CHEN, Jie, Shenzhen, Guangdong 518055 (CN); XIAO, Tianxia, Shenzhen, Guangdong 518055 (CN)
(74) Representative: Fabry, Bernd
(86) International application number: PCT/CN2023/090364
(87) International publication number: WO 2024/221184

(57) **Abstract**

The present invention discloses polypeptides and related substances against hair loss and dermatitis, and the uses thereof. Particularly, disclosed is the use of an osteocalcin-derived peptide, and homologous polypeptides, polypeptide-derived peptides or derivatives of the polypeptides and polypeptide-derived peptides, which use the osteocalcin-derived peptide as a skeleton and which are derived from different species for the manufacture of a medicament for treating hair follicle recession and/or hair loss caused by psychological stress, seborrheic hair follicle recession and/or hair loss, acne and/or dermatitis caused by psychological stress and seborrheic acne and/or dermatitis. Further disclosed are the use of substances capable of binding to a GPR158 receptor and modulating its activity for the manufacture of medicaments, medical cosmetology products, cosmetics, health-care products, foods and additives for preventing and/or treating hair follicle recession, hair loss, acne and dermatitis, and a method for preparing a related hair loss model on the basis of the discovery of the GPR158 receptor.

## Description

### TECHNICAL FIELD

The present disclosure belongs to the field of biomedicine, and relates to polypeptides and related substances against hair loss and the like, and the uses thereof.

### BACKGROUND

Hair loss (loss of hair) refers to a phenomenon of hair shedding, and is classified into physiological hair loss and pathological hair loss. The physiological hair loss refers to the normal shedding of hair during a catagen phase and a telogen phase to maintain a normal hair density. In contrast, the pathological hair loss refers to abnormal or excessive shedding of hair.

According to data from the Report on Hair Loss Population in China released in 2017, the current hair loss population in China is approximately 200 million, and this number is increasing at an annual rate of at least 15%. The majority of the hair loss population are male, amounting to approximately 130 million.

The hair loss is attributed to a number of factors, including genetic predisposition, immune dysfunction, excessive psychological stress, staying up late, medications (such as chemotherapy-induced alopecia), environmental influences, and endocrine disorders.

According to etiology, the hair loss is classified into psychological stress-induced alopecia, androgenetic (seborrheic) alopecia, pathological alopecia, physical/chemical-induced alopecia, nutritional alopecia, obesity-related alopecia, hereditary alopecia, and the like. Currently, two medicaments for treating the hair loss have been approved by the U.S. Food and Drug Administration (FDA), including Minoxidil and Finasteride. Of the two medicaments, Minoxidil, as a topical scalp drug, exhibits an initial excessive shedding phase, a prolonged period for efficacy, and certain adverse effects; and Finasteride, as an oral medicament, poses significant risks to female fertility and demonstrates no efficacy in postmenopausal women, such that this medicament is not recommended for female patients. Therefore, there exists a clinical need for development of interventional medicaments that are safer, more effective, and applicable to diverse populations and various types of hair loss.

The androgenetic alopecia (AGA), classified into male-pattern hair loss (MPHL) and female-pattern hair loss (FPHL), is induced by genetic predisposition and androgens. Differences in sex hormones between male patients and female patients result in significant distinctions between the MPHL and the FPHL, which are mainly reflected in etiology, diagnosis, and treatment aspects. As androgen levels are high in the male patient, testosterone is converted to dihydrotestosterone (DHT) by 5α-reductase after reaching hair follicles. The DHT induces hair follicle atrophy, hair gradually thins, and hair loss occurs in genetically predisposed male patients. The hair loss is mainly manifested as a receding hairline, M-shaped hair loss, and eventual baldness. In the female patient, androgen levels are low and estrogens inhibit androgens. However, when a hormonal equilibrium is disrupted or estrogen levels decline after a middle age, the hair loss may occur due to insufficient estrogen secretion or excessive androgens. The hair loss mainly presents a progressive form, is clinically characterized by progressive hair follicle miniaturization, and exhibits gradual thinning and sparseness of hair at vertexes of heads.

Roles of androgens in pathogenesis of the MPHL are basically clear, but those in pathogenesis of the FPHL are not clear. In addition to the androgens, the etiology of the FPHL may involve non-androgen-dependent pathways, such as chronic low-grade scalp inflammation, androgen insensitivity syndrome, estrogen deficiency, thyroid dysfunction, and metabolic syndrome [referenced from the "Chinese Expert Consensus on the Diagnosis and Treatment of Female Androgenetic Alopecia (2022)"].

### SUMMARY

In view of the therapeutic bottlenecks for hair loss mentioned in the background above, an objective of the present disclosure is to provide the uses of polypeptides and related substances against hair loss and the like.

In one aspect, the present disclosure provides use of a polypeptide, polypeptide-derived peptides, or derivatives of the polypeptide and polypeptide-derived peptides for the manufacture of a medicament for treating hair follicle recession and/or hair loss caused by psychological stress, seborrheic hair follicle recession and/or hair loss, acne and/or dermatitis caused by psychological stress and seborrheic acne and/or dermatitis,.

The polypeptide is selected from an osteocalcin-derived peptide and homologous polypeptides which use the osteocalcin-derived peptide as a skeleton and which are derived from different species, with sequences as follows:
YLYQWLGAPVPYPDPLEPR SEQ ID No.1
YLGASVPSPDPLEPT SEQ ID No.2
LDPGLGAPAPYPDPLEPR SEQ ID No.3
NYNRFYNARAAPTPDPLEPL SEQ ID No.4
SNLRNAVFGTPVRDPLESK SEQ ID No.5
AGAVTAADLSLTQLESL SEQ ID No.6
YAQDSGVAGAPPNPLEAQ SEQ ID No.7
SDFYER YFMHYKTPMEQM SEQ ID No.8
in these sequences, SEQ ID NO.1 represents a human osteocalcin-derived peptide, SEQ ID NO.2 represents an osteocalcin-derived peptide derived from a mouse, SEQ ID NO.3 represents an osteocalcin-derived peptide derived from a sheep of a livestock species (the sequence is identical to those derived from an otter and a badger), SEQ ID NO.4 represents an osteocalcin-derived peptide derived from a lizard of a reptile species, SEQ ID NO.5 represents an osteocalcin-derived peptide derived from a water frog of an amphibian species, SEQ ID NO.6 represents an osteocalcin-derived peptide derived from a Wuchang fish of a bony fish species, SEQ ID NO.7 represents an osteocalcin-derived peptide derived from a chicken of a poultry species, and SEQ ID NO.8 represents an osteocalcin-derived peptide derived from a mandarin duck of a bird species (the sequence is identical to that derived from a swan goose).

Given functional similarities of the osteocalcin-derived peptides derived from different species, the osteocalcin-derived peptides include osteocalcin-derived peptides derived from mammal, reptile, amphibian, bony fish, poultry, and bird species.

The polypeptide-derived peptides are conserved fragments derived from osteocalcin of different biological species, the polypeptide-derived peptides are located at the same conserved fragment position as any one of the sequences set forth in SEQ ID NO.1-8, and the different biological species are selected from mammal, reptile, amphibian, bony fish, poultry, and bird species; the polypeptide-derived peptides have sequences X₁-X₂-...Xₙ, where X represents an amino acid, and n represents any integer from 8 to 25; and the immediately adjacent upstream and downstream sequences of the polypeptide-derived peptides in osteocalcin are both convertase cleavage sites;
the derivatives are products obtained by terminal or side chain modification of the above polypeptide or polypeptide-derived peptide, or products obtained by linkage to tags for polypeptide or protein detection or purification, or products obtained by isotope labeling modification; and
the products obtained by modification are selected from products obtained by fluorophore modification, products obtained by phosphorylation modification, products obtained by disulfide bond-based cyclization modification, products obtained by biotin labeling modification, products obtained by photosensitizer modification, products obtained by azide modification, products obtained by polyethylene glycol (PEG) modification, products obtained by methylation modification, products obtained by fluorescence quenching group modification, products obtained by protein conjugation modification, or products obtained by small molecule compound modification; or products obtained by aminylation modification, amidation modification, hydroxylation modification, carboxylation modification, carbonylation modification, alkylation modification, acetylation modification, esterification modification, or glycosylation modification.

Further, the terminal modification is selected from N-terminal acetylation modification and C-terminal amidation modification of the polypeptide.

Further, the side chain modification is selected from modification on an R group of an amino acid side chain in the polypeptide.

Further, fluorescent dye used in the fluorophore modification is selected from aminomethylcoumarin acetic acid (AMCA), fluorescein isothiocyanate (FITC), Rhodamine, Cy3, Cy5, Cy5.5, Cy7, aggregation-induced emission (AIE), and indocyanine green (ICG), and this modification can be used for fluorescence detection.

Further, the phosphorylation modification is selected from one or more of p-Ser, p-Thr, and p-Tyr.

Further, the glycosylation modification is introduced at one or more amino acid residues selected from Ser, Asn, Thr, and Tyr.

Further, the nitrosylation modification is introduced at the Tyr.

Further, biotin used in the biotin labeling is selected from D-biotin, biotin hydrazide, photobiotin, and biotin-dUTP.

Further, the photosensitizer modification can be used to prepare photosensitive formulations.

Further, the azide modification can be used for secondary coupling reactions.

Further, the PEG modification can be used to prepare drug carriers.

Further, an isotope used in the isotope labeling is selected from one or more of ¹³C, ¹⁴C, ¹⁴N, ¹⁵N, ²H, ³H, ¹⁸O, ³²P, ³²S, ³⁴S, ³⁵S, ³⁶S, ³⁵Cl, ³⁷Cl, ¹²⁵I, and ¹³¹I.

The polypeptide and derivatives which use the polypeptide as a skeleton may be autonomously synthesized under general chemical laboratory conditions. A recombinant protein containing the above polypeptide is expressed via genetic engineering, or the polypeptide is industrially synthesized by a commercial reagent company. The polypeptide is synthesized by using a solid-phase method, where different amino acids on a resin are directionally synthesized into an amino acid chain via a condensation reaction. Alternatively, the polypeptide comes from extracts isolated from tissues or organs of different species. The derivatives of the polypeptide are prepared by labeling with modifying groups on the amino acids which are linked.

Further, the hair follicle recession and/or hair loss caused by psychological stress refers to hair follicle recession and/or hair loss of a female or male subject caused by psychological stress;
the seborrheic hair follicle recession and/or hair loss refers to seborrheic hair follicle recession and/or hair loss of a male subject;
the acne and/or dermatitis caused by psychological stress refers to acne and/or dermatitis of a female or male subject caused by psychological stress; and
the seborrheic acne and/or dermatitis refers to seborrheic acne and/or dermatitis of a male subject.

In another aspect, the present disclosure provides an effect of the above polypeptide binding to a receptor thereof and modulating its activity.

Further, the receptor mainly refers to GPR158.

In another aspect, the present disclosure provides use of substances capable of binding to a GPR158 receptor and modulating its activity for the manufacture of a medicament for treating hair follicle recession and/or hair loss caused by psychological stress, seborrheic hair follicle recession and/or hair loss, acne and/or dermatitis caused by psychological stress and seborrheic acne and/or dermatitis.

Further, the substances capable of binding to a GPR158 receptor and modulating its activity include the above polypeptide.

Further, the substances capable of binding to a GPR158 receptor and modulating its activity, in addition to the above polypeptide, include inorganic ions such as cations/metal ions, anions, and hydrogen ions; organic molecules such as polypeptides and small molecule compounds; proteins and biological macromolecules such as enzymes, and peptides that bind to the receptor; and antibodies, siRNAs, shRNAs, antisense RNA and DNAzymes, or combinations thereof, and expression vectors including siRNAs, shRNAs, and antisense molecules.

In another aspect, the present disclosure provides a method for preparing an animal model of hair loss, where the method includes knocking out a GPR158 receptor gene in an animal, or treating the animal with a GPR158 receptor inhibitor.

In another aspect, the present disclosure provides of th of the above polypeptide and derivatives thereof, and substances capable of binding to a GPR158 receptor and modulating its activity e above polypeptide and derivatives thereof, and substances capable of binding to a GPR158 receptor and modulating its activity use of the above polypeptide and derivatives thereof, and substances capable of binding to a GPR158 receptor and modulating its activity, in preparing medical cosmetology products, cosmetics, health-care products, foods and additives for preventing and/or treating hair follicle recession, hair loss, acne and dermatitis.

Further, the hair loss includes psychological stress-induced hair follicle regression and/or hair loss, and androgenetic hair follicle regression and/or hair loss of a female subject and a male subject;
further, the prevention and/or treatment of the hair loss refers to promoting hair growth.
further, the acne includes psychological stress-induced acne and androgenetic acne of a female subject and a male subject; and
further, the dermatitis includes psychological stress-induced dermatitis and androgenetic dermatitis of a female subject and a male subject.

In another aspect, the present disclosure provides use of the above polypeptide and derivatives thereof, or substances capable of binding to a GPR158 receptor and modulating its activity for the manufacture of detection reagents for targeting brains, adrenal glands, female gonads (ovary and uterus), and male gonads (testis and epididymis),.

In another aspect, the present disclosure provides use of the above polypeptide and derivatives thereof, or substances capable of binding to a GPR158 receptor and modulating its activity for the manufacture of a medicament for inhibiting elevation of corticosteroids or glucocorticoids.

In another aspect, the present disclosure provides use of the above polypeptide and derivatives thereof, or substances capable of binding to a GPR158 receptor and modulating its activity for the manufacture of a medicament for inhibiting inflammatory responses induced by elevated corticosteroids, glucocorticoids, or cortisols.

In another aspect, the present disclosure provides use of substances capable of binding to a GPR158 receptor and inhibiting functions for the manufacture of products for hair loss-related research models,.

In a further aspect, the present disclosure provides a medicament for preventing and/or treating related symptoms such as hair follicle regression, hair loss, acne, and dermatitis, where the medicament includes a polypeptide, polypeptide-derived peptides, or derivatives of the polypeptide and polypeptide-derived peptides, and a conjugate that combines a GPR158 receptor agonist, an antagonist, and active ingredients;
the medicament may include one or more pharmaceutically acceptable carriers;
the carriers are preferably selected from a diluent, an excipient, a filler, a binder, a wetting agent, a disintegrating agent, an absorption enhancer, an adsorbent carrier, a surfactant, a lubricant, and the like;
the medicament may be further formulated into various forms such as a drop, a tablet, a granule, a capsule, an oral liquid, an inhalation liquid, a topical liquid, a spray, a microneedle, or an injectable, and various dosage forms may be prepared according to conventional methods in the pharmaceutical field; and
administration modes of the medicament include oral administration, topical contact, administration as a suppository, or intravenous, intraperitoneal, intramuscular, intralesional, intrathecal, intranasal, or subcutaneous administration to a subject. Administration routes include parenteral routes such as intravenous, intramuscular, intraarterial, intradermal, subcutaneous, intraperitoneal, intraventricular, and intracranial; and transmucosal routes such as buccal, sublingual, palatal, gingival, intranasal, vaginal, rectal, or transdermal. Other delivery modes include, but are not limited to, liposomal formulations, intravenous infusions, and transdermal patches.

In another aspect, the present disclosure provides a method for treating or preventing hair follicle recession and/or hair loss caused by psychological stress, seborrheic hair follicle recession and/or hair loss, acne and/or dermatitis caused by psychological stress and seborrheic acne and/or dermatitis, where the method includes a step of administering to a subject a polypeptide, polypeptide-derived peptides, or derivatives of the polypeptide and polypeptide-derived peptides, or substances capable of binding to a GPR158 receptor and modulating its activity;
the polypeptide is selected from an osteocalcin-derived peptide and homologous polypeptides which use the osteocalcin-derived peptide as a skeleton and which are derived from different species, with sequences as follows:
   YLYQWLGAPVPYPDPLEPR SEQ ID No.1
   YLGASVPSPDPLEPT SEQ ID No.2
   LDPGLGAPAPYPDPLEPR SEQ ID No.3
   NYNRFYNARAAPTPDPLEPL SEQ ID No.4
   SNLRNAVFGTPVRDPLESK SEQ ID No.5
   AGAVTAADLSLTQLESL SEQ ID No.6
   YAQDSGVAGAPPNPLEAQ SEQ ID No.7
   SDFYER YFMHYKTPMEQM SEQ ID No.8
the polypeptide-derived peptides are conserved fragments derived from osteocalcin of different biological species, the polypeptide-derived peptides are located at the same conserved fragment position as any one of the sequences set forth in SEQ ID NO.1-8, and the different biological species are selected from mammal, reptile, amphibian, bony fish, poultry, and bird species; the polypeptide-derived peptides have sequences X₁-X₂-...Xₙ, where X represents an amino acid, and n represents any integer from 8 to 25; and the immediately adjacent upstream and downstream sequences of the polypeptide-derived peptides in osteocalcin are both convertase cleavage sites;
the derivatives of the polypeptide are products obtained by terminal or side chain modification of the polypeptide or polypeptide-derived peptide, or products obtained by linkage to tags for polypeptide or protein detection or purification, or products obtained by isotope labeling modification;
further, the products obtained by modification are selected from products obtained by fluorophore modification, products obtained by phosphorylation modification, products obtained by disulfide bond-based cyclization modification, products obtained by biotin labeling modification, products obtained by photosensitizer modification, products obtained by azide modification, products obtained by polyethylene glycol (PEG) modification, products obtained by methylation modification, products obtained by fluorescence quenching group modification, products obtained by protein conjugation modification, or products obtained by small molecule compound modification; or products obtained by aminylation modification, amidation modification, hydroxylation modification, carboxylation modification, carbonylation modification, alkylation modification, acetylation modification, esterification modification, or glycosylation modification;
further, the terminal modification is selected from N-terminal acetylation modification and C-terminal amidation modification of the polypeptide;
further, the side chain modification is selected from modification on an R group of an amino acid side chain in the polypeptide;
further, fluorescent dye used in the fluorophore modification is selected from AMCA, FITC, Rhodamine, Cy3, Cy5, Cy5.5, Cy7, AIE, and ICG, and this modification can be used for fluorescence detection;
further, the phosphorylation modification is selected from one or more of p-Ser, p-Thr, and p-Tyr;
further, the glycosylation modification is introduced at one or more amino acid residues selected from Ser, Asn, Thr, and Tyr;
further, the nitrosylation modification is introduced at the Tyr;
further, biotin used in the biotin labeling is selected from D-biotin, biotin hydrazide, photobiotin, and biotin-dUTP;
further, the photosensitizer modification can be used to prepare photosensitive formulations;
further, the azide modification can be used for secondary coupling reactions;
further, the PEG modification can be used to prepare drug carriers; and
further, an isotope used in the isotope labeling is selected from one or more of ¹³C, ¹⁴C, ¹⁴N, ¹⁵N, ²H, ³H, ¹⁸O, ³²P, ³²S, ³⁴S, ³⁵S, ³⁶S, ³⁵Cl, ³⁷Cl, ¹²⁵I, and ¹³¹I.

Further, the step of administering a polypeptide or derivatives thereof to a subject includes administering the polypeptide or derivatives thereof via an oral, intravenous, intramuscular, transdermal, or transmucosal route.

The present disclosure has the following beneficial effects:
(1) The polypeptide and derivatives which use the polypeptide as a skeleton provided by the present disclosure may resist hair follicle recession, and/or hair loss, and/or acne, and/or dermatitis caused by psychological stress, and/or androgens, and/or seborrheic factors.
(2) The candidate polypeptides and derivatives which use the polypeptide as a skeleton provided by the present disclosure are easy to synthesize and modify, with low costs and broad application prospects.
(3) The candidate polypeptides and derivatives which use the polypeptide as a skeleton provided by the present disclosure provide a comprehensive strategy for preparing diagnostic and therapeutic compounds or drugs for practical applications.
(4) The candidate polypeptides and derivatives which use the polypeptide as a skeleton provided by the present disclosure are suitable for applications to hair loss and related symptoms.
(5) The present disclosure demonstrates relationships between GPR158 and hair loss as well as hair follicles, and the relationships may be used for establishing animal models of hair loss or dermatitis and serve as research and observation tools in experimental animals for modeling of hair loss diseases.
(6) Respective docking of the polypeptides from different species with the human GPR158 receptor indicates that all the polypeptides exhibit a high binding affinity, which expands types of active polypeptides available for treating conditions such as hair loss and dermatitis.

### BRIEF DESCRIPTION OF THE DRAWINGS

FIG. 1 is an effect diagram of *in vivo* targeting of a mouse-derived polypeptide (mO15-OCG) linked to a dye indocyanine green (ICG) toward brains, ovaries, and uteruses of female mice. The figure illustrates fluorescence signal distribution and results in the brains, ovaries, and uteruses of the mice in three groups: a PBS group, an ICG group, and an ICG-linked polypeptide group.
FIG. 2 is an effect diagram of *in vivo* targeting of a Cy5-labeled mouse-derived polypeptide (Cy5-mO15) toward adrenal glands of female mice. The figure illustrates fluorescence signal distribution and results in the adrenal glands of the mice in an mO15 group and a control group.
FIG. 3 is an effect diagram of *in vivo* targeting of a Cy5-labeled mouse-derived polypeptide (Cy5-mO15) toward brains, gonads, and adrenal glands of male mice. The figure illustrates fluorescence signal distribution and results in the adrenal glands, brains, testis, and epididymis of the mice in three groups: a PBS group, a control polypeptide group, and an mO15 group.
FIG. 4 illustrates expression and localization results of a polypeptide GPR158 receptor in human brain, ovary, testis, and skin tissues, as summarized from single-cell sequencing results of various human tissues in The Human Protein Atlas database.
FIG. 5 illustrates immunofluorescence staining results for GPR158 in hypothalami, pituitary glands, and ovaries of mice. The figure shows specific expression of GPR158 in subventricular regions of the hypothalami.
FIG. 6A illustrates the results of molecular docking simulations between homologous sequence polypeptides from different species and the human GPR158 receptor. These homologous sequence polypeptides share over 60% sequence identity and are all capable of binding to the extracellular domain of the human GPR158 receptor .
FIG. 6B illustrates all interaction scores, all of which are above 20, indicating a strong binding affinity between different polypeptides and the human GPR158 receptor.
FIG. 7 illustrates endocytosis of osteocalcin and an osteocalcin-derived peptide by a HeLa cell line highly expressing a GPR158 receptor. The figure shows results of human osteocalcin (Ocn49) and an osteocalcin-derived peptide (Ocn19) binding to and being internalized by HeLa cells highly expressing a GPR158 receptor. Red represents a polypeptide and polypeptide-derived peptides located in a cytoplasm, while blue represents cell nuclei stained with 4',6-diamidino-2-phenylindole (DAPI).
FIG. 8A shows binding rates of the homologous polypeptides from different species to HeLa cells highly expressing a GPR158 receptor.
FIG. 8B shows intensities of calcium signals generated after binding of the homologous polypeptides from different species to the HeLa cells highly expressing a GPR158 receptor.
FIG. 9A shows hair growth conditions of cervicodorsal regions of mice in a corticosteroid Dexamethasone (DXM) model group, a glucocorticoid receptor agonist Prednisone model group, and a polypeptide treatment group.
FIG. 9B shows scoring criteria for mouse skin color.
FIG. 9C shows results of quantifying the results in FIG. 9A according to the criteria in FIG. 9B.
FIG. 10A shows hematoxylin and eosin (HE) staining results of the cervicodorsal skins of mice in a Dexamethasone group and a polypeptide treatment group in a stress-induced hair loss model.
FIG. 10B shows count results of hair follicles in the skins according to morphological observations in FIG. 10A.
FIG. 10C shows expression results of cortisol synthesis-related enzyme genes 3betaHSD, Cyp21, and Cyp11B1, a testosterone synthesis-related enzyme gene 17beta-HSD3, an estrogen synthesis-related enzyme gene Cyp19, and a glucocorticoid receptor gene GR in the skin of mice from the stress-induced hair loss model.
FIG. 11A shows hair growth conditions of cervicodorsal regions of mice in an androgen group, and an androgen + polypeptide treatment group.
FIG. 11B shows the scoring criteria for mouse skin color in the androgen group, and the androgen + polypeptide treatment group.
FIG. 11C shows results of quantifying the results in FIG. 11A according to the criteria in FIG. 11B.
FIG. 12A shows HE staining results of the cervicodorsal skins of the mice in a control group, a DHT treatment group, and a polypeptide treatment group in an androgenetic(seborrheic) hair loss model.
FIG. 12B shows count results of hair follicles in the skins according to morphological observations in FIG. 12A.
FIG. 12C shows expression results of a Wnt signaling pathway regulatory protein β-catenin, a Wnt signaling pathway antagonist DKK1, inflammatory factors TGF-β and IL-6, and an androgen receptor (AR) in the skins of mice from the androgenetic(seborrheic) hair loss model..
FIG. 13 illustrates protein expression (by immunofluorescence) results of an AR in cervicodorsal skins of mice in an androgenetic (seborrheic) hair loss model. Green represents AR immunofluorescence staining, and blue represents cell nuclei stained with DAPI.
FIG. 14 illustrates gene expression results (by quantitative real-time polymerase chain reaction (PCR)) of sebaceous gland-related molecular markers in cervicodorsal skins of mice in an androgenetic (seborrheic) hair loss model. LPL represents lipoprotein lipase, PPARγ promotes adipocyte differentiation, and FABP3/4 represents a fatty acid-binding protein.
FIG. 15A shows hair growth conditions of cervicodorsal regions of wild-type mice and GPR158KO female mice at the same age(10-week-old).
FIG. 15B shows scoring criteria for mouse skin color.
FIG. 15C shows results of quantifying the results in FIG. 15A according to the criteria in FIG. 15B.
FIG. 16A shows HE staining results of cervicodorsal skins of wild-type mice and GPR158KO female mice at the same age(10-week-old).
FIG. 16B shows count results of hair follicles in the skins according to morphological observations in FIG. 16A.
FIG. 16C shows expression results of 5α-reductase (an enzyme converting testosterone to DHT), cortisol synthesis-related enzyme genes 3betaHSD, Cyp21, and Cyp11B1, a testosterone synthesis-related enzyme gene 17betaHSD3, an estrogen synthesis-related enzyme gene Cyp19, and a glucocorticoid receptor gene GR in the skins.
FIG. 17 illustrates identification results of human primary dermal papilla cells. Green represents fibronectin.
FIG. 18A illustrates alleviating effects of homologous sequence polypeptides from human, mouse, and livestock species on changes in cell-related molecular markers after inducing a psychological stress model *in vitro* using human primary dermal papilla cells through cortisol treatment.
FIG. 18B shows alleviating effects of homologous sequence polypeptides from human, reptile, amphibian, and fish species on changes in cell-related molecular markers after inducing a psychological stress model *in vitro* using human primary dermal papilla cells through cortisol treatment.
FIG. 18C shows alleviating effects of homologous sequence polypeptides from human, poultry, and bird species species on changes in cell-related molecular markers after inducing a psychological stress model *in vitro* using human primary dermal papilla cells through cortisol treatment.

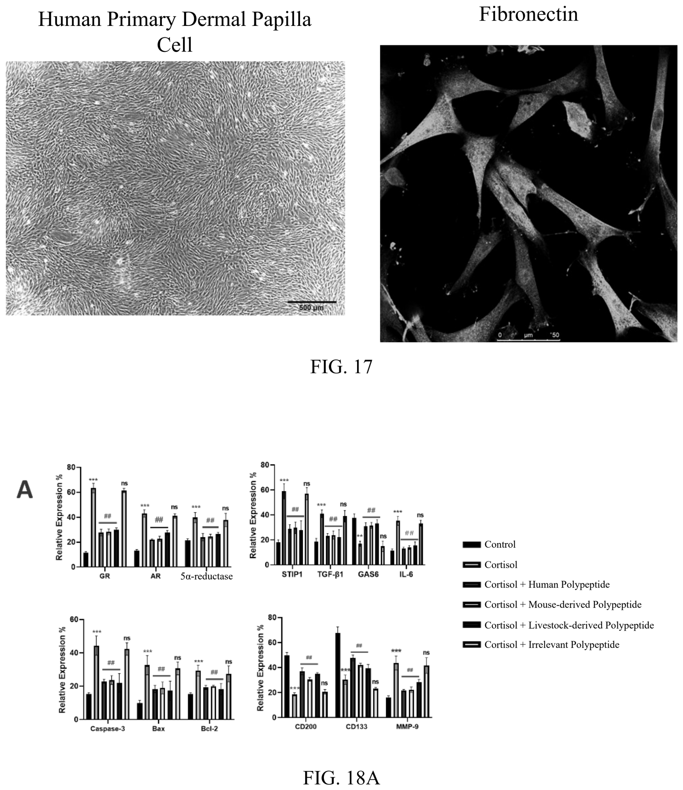

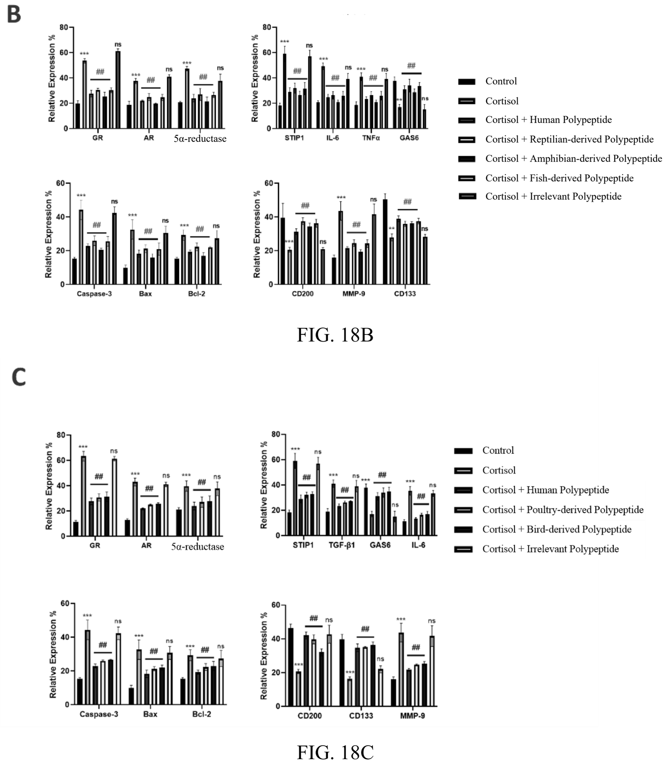

In FIGS. 18A, 18B and 18C, AR represents an androgen receptor; 5α-reductase promotes conversion of testosterone to DHT; STIP1 represents a psychological stress-induced phosphoprotein; GAS6 represents a hair growth marker; TGF-β1 and IL-6 represent inflammatory factors; Caspase-3, Bax, and Bcl-2 represent hair follicle cell apoptosis markers; CD200 and CD133 represent hair follicle stem cell markers; and MMP-9 represents a hair follicle regression marker.

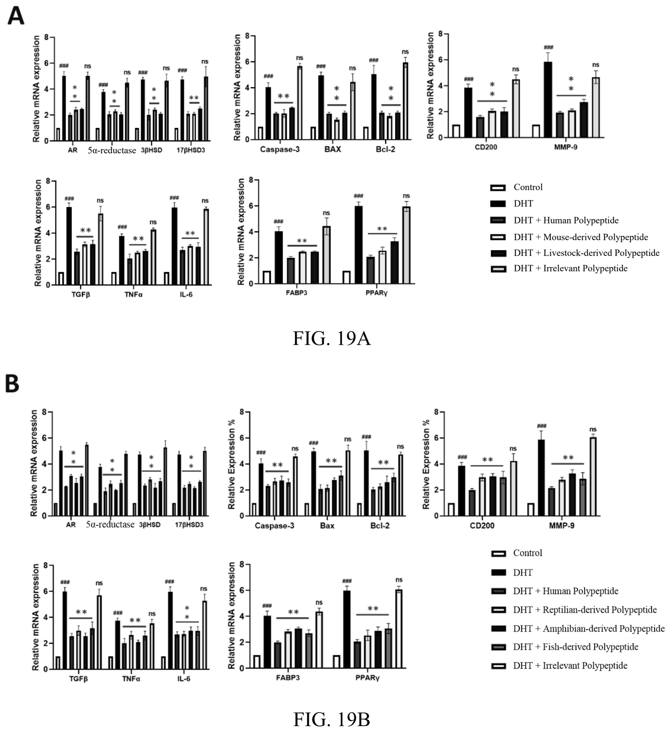

FIG. 19A shows alleviating effects of homologous sequence polypeptides from human, mouse, and livestock species on changes in cell-related molecular markers after inducing an androgenetic/seborrheic model *in vitro* using human primary dermal papilla cells through DHT treatment.
FIG. 19B shows alleviating effects of homologous sequence polypeptides from human, reptile, amphibian, and fish species on changes in cell-related molecular markers after inducing an androgenetic/seborrheic model *in vitro* using human primary dermal papilla cells through DHT treatment.

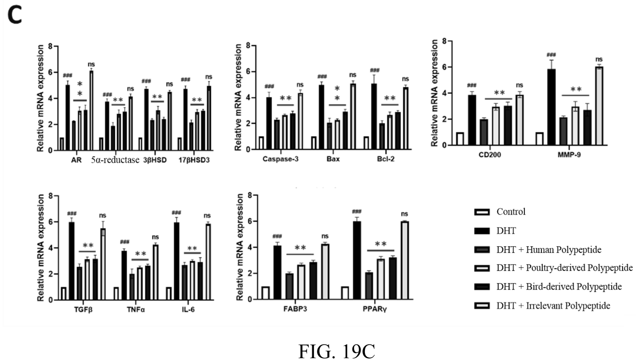

FIG. 19C shows alleviating effects of homologous sequence polypeptides from human, poultry, and bird species on changes in cell-related molecular markers after inducing an androgenetic/seborrheic model *in vitro* using human primary dermal papilla cells through DHT treatment.

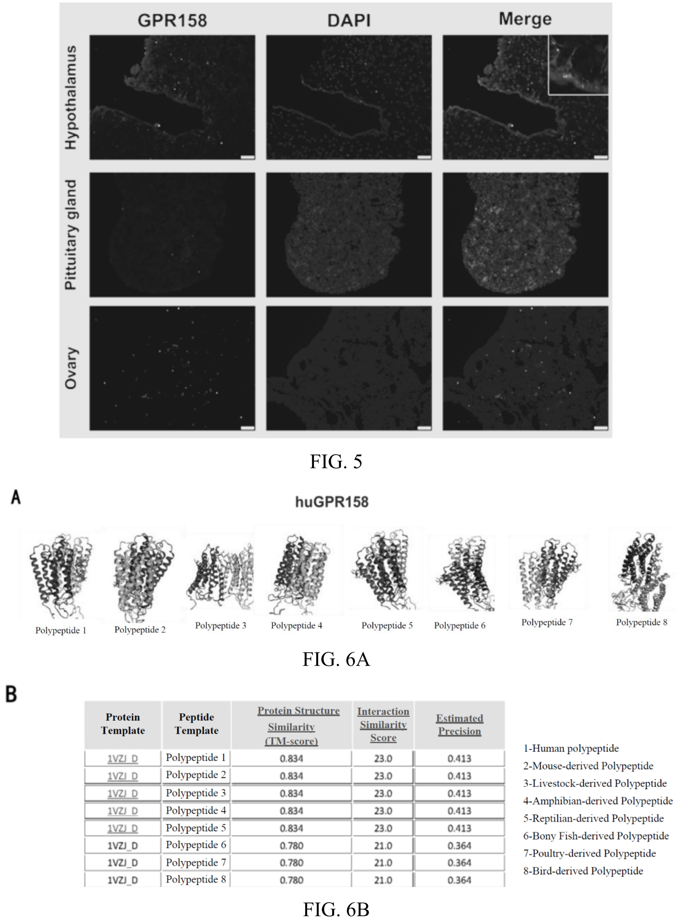

In FIGS. 19A, 19B and 19C, 5α-reductase promotes conversion of testosterone to DHT; 3β-HSD and 17β-HSD are hormone synthesis-related enzymes; Caspase-3, Bax, and Bcl-2 represent hair follicle cell apoptosis markers; CDCD200 represents a hair follicle stem cell marker; MMP-9 represents a hair follicle regression marker; TGF-β, TNFα, and IL-6 represent inflammatory factors; FABP3 represents a fatty acid-binding protein; and PPARγ promotes adipocyte differentiation.

### DETAILED DESCRIPTIONS OF THE EMBODIMENTS

To better understand the contents of the present disclosure, the contents of the present disclosure are further illustrated below in conjunction with specific embodiments. However, the protection scope of the present disclosure is not limited to the following embodiments.

### Terms

Hair follicle recession and/or hair loss caused by psychological stress refer/refers to a condition that psychological stress hormones released by adrenal glands due to chronic psychological stress, i.e., corticosteroids, lead to a prolonged telogen phase of hair follicles, impair activation of hair follicle stem cells, and result in hair regeneration disorders.

Seborrheic hair follicle recession and/or hair loss refer/refers to a condition that androgens promote sebaceous glands to secrete excessive sebum which accumulates around hair follicles to induce hair follicle miniaturization, ultimately leading to hair loss.

Acne and/or dermatitis caused by psychological stress refer/refers to a condition that psychological stress, by acting on a hypothalamic-pituitary-adrenal (HPA) axis, promotes release of psychological stress hormones and pro-inflammatory mediators, which stimulates sebaceous glands and hair follicles, and leads to increased skin inflammation.

Seborrheic acne and/or dermatitis refer/refers to acne and/or dermatitis resulting from inflammatory responses triggered by accumulation of excessive sebum in hair follicles.

In the present disclosure, both polypeptides and polypeptide-derived peptides are conserved sequence polypeptides located in osteocalcin from different biological species, where SEQ ID NO.1-8 are sourced as follows: SEQ ID NO.1 represents a human osteocalcin-derived peptide, SEQ ID NO.2 represents an osteocalcin-derived peptide derived from a mouse, SEQ ID NO.3 represents an osteocalcin-derived peptide derived from a sheep of a livestock species (the sequence is identical to those derived from an otter and a badger), SEQ ID NO.4 represents an osteocalcin-derived peptide derived from a lizard of a reptile species, SEQ ID NO.5 represents an osteocalcin-derived peptide derived from a water frog of an amphibian species, SEQ ID NO.6 represents an osteocalcin-derived peptide derived from a Wuchang fish of a bony fish species, SEQ ID NO.7 represents an osteocalcin-derived peptide derived from a chicken of a poultry species, and SEQ ID NO.8 represents an osteocalcin-derived peptide derived from a mandarin duck of a bird species (the sequence is identical to that derived from a swan goose).

The polypeptide-derived peptides are conserved fragments derived from osteocalcin of different biological species except the above SEQ ID NO.1-8, the polypeptide-derived peptides are located at the same conserved fragment position as any one of the sequences set forth in SEQ ID NO.1-8, and the different biological species are selected from mammal, reptile, amphibian, bony fish, poultry, and bird species; the polypeptide-derived peptides have sequences X₁-X₂-...Xₙ, where X represents an amino acid, and n represents any integer from 8 to 25; and the immediately adjacent upstream and downstream sequences of the polypeptide-derived peptides in osteocalcin are both convertase cleavage sites.

In the osteocalcin, there are two conserved sequences. The polypeptides and the polypeptide-derived peptides in the present disclosure are both conserved sequences near N-terminals.

### Example 1: Preparation of polypeptide sequences

The polypeptide sequences were synthesized artificially.

The polypeptide sequences are as follows:
YLYQWLGAPVPYPDPLEPR SEQ ID No.1 (Human, Ocn19)
YLGASVPSPDPLEPT SEQ ID No.2 (Mouse, mO15/OC15/mOC15/Ocn15)
LDPGLGAPAPYPDPLEPR SEQ ID No.3 (Livestock species-polypeptide)
NYNRFYNARAAPTPDPLEPL SEQ ID No.4 (Amphibian species-polypeptide)
SNLRNAVFGTPVRDPLESK SEQ ID No.5 (Reptile species-polypeptide)
AGAVTAADLSLTQLESL SEQ ID No.6 (Bony fish species-polypeptide)
YAQDSGVAGAPPNPLEAQ SEQ ID No.7 (Poultry species-polypeptide)
SDFYER YFMHYKTPMEQM SEQ ID No.8 (Bird species-polypeptide)

The above polypeptides were synthesized by using conventional solid-phase synthesis or liquid-phase synthesis methods. In these methods, a solid-phase polypeptide synthesis method was employed to enable a reaction from an amino acid at a C-terminal to an amino acid at an N-terminal. Through resin activation, amino acid linkage, deprotection by washing, and detection, amino acids were linked one by one. The reaction mixture was then precipitated with an excess of ether and centrifuged to obtain crude peptides. After purification by high-performance liquid chromatography (HPLC), the crude peptides were analyzed by mass spectrometry, and rapidly frozen and lyophilized in liquid nitrogen for later use.

In these sequences, SEQ ID NO.1 represents a human osteocalcin-derived peptide, SEQ ID NO.2 represents an osteocalcin-derived peptide derived from a mouse, SEQ ID NO.3 represents an osteocalcin-derived peptide derived from a sheep of a livestock species (the sequence is identical to those derived from an otter and a badger), SEQ ID NO.4 represents an osteocalcin-derived peptide derived from a lizard of a reptile species, SEQ ID NO.5 represents an osteocalcin-derived peptide derived from a water frog of an amphibian species, SEQ ID NO.6 represents an osteocalcin-derived peptide derived from a Wuchang fish of a bony fish species, SEQ ID NO.7 represents an osteocalcin-derived peptide derived from a chicken of a poultry species, and SEQ ID NO.8 represents an osteocalcin-derived peptide derived from a mandarin duck of a bird species (the sequence is identical to that derived from a swan goose).

Many proteins and hormones exhibit a high homology between humans and animals. For instance, bovine insulin and porcine insulin have been used for treating diabetic patients; pregnant mare serum gonadotropin (PMSG) has also been employed for superovulation in both humans and animals; a chemical structure of oxytocin is identical in humans and most mammals; and since isolation of gonadotropin-releasing hormone (GnRH) from porcine and ovine brains in 1971, at least 28 types have been identified in a GnRH family to date, among which 15 types are derived from vertebrates and 13 types are derived from invertebrates. With the exception of octopus GnRH (octo GnRH), all GnRH peptides are composed of ten amino acids, with significant conservation in molecular lengths and partial amino acid sequences.

In view of the homology and functional similarities of the polypeptides and hormones across different species, sequences derived from the same species as the above polypeptides or sequences or having a high homology with the above polypeptides are likely to achieve the same effects.

### Example 2: Preparation of polypeptide probe

This example provides a polypeptide probe, which is prepared by combining the polypeptide obtained in Example 1 with ICG or Cy5 via an organic chemical reaction.

A specific method involves either commissioned synthesis or in-house synthesis. In this example, the probe was prepared by linking the polypeptide to ICG via a click chemistry method, where universal dibenzocyclooctyne (DBCO) served as a linker. The ICG, having activated functional groups including amino (NH₂), carboxyl (COOH), N-hydroxysuccinimide ester (NHS), maleimide (MAL), sulfhydryl (SH), azide (N₃), and alkyne (ALK), was conjugated with the polypeptide prepared in Example 1 to obtain the polypeptide-ICG probe.

### Example 3: Detection of targeting capability of polypeptide-ICG/Cy5 probe to brains, ovaries, and uteruses of female mice

Adult female mice were intravenously injected via tail veins with the following: the polypeptide-ICG probe solution prepared in Example 2/Cy5-labeled polypeptide, an ICG solution (2 mM)/control polypeptide (a scrambled, irrelevant polypeptide), and a phosphate buffered saline (PBS). Each mouse received an injection volume of 100 µL. After 24 hours, detection was performed using a small animal imaging instrument.

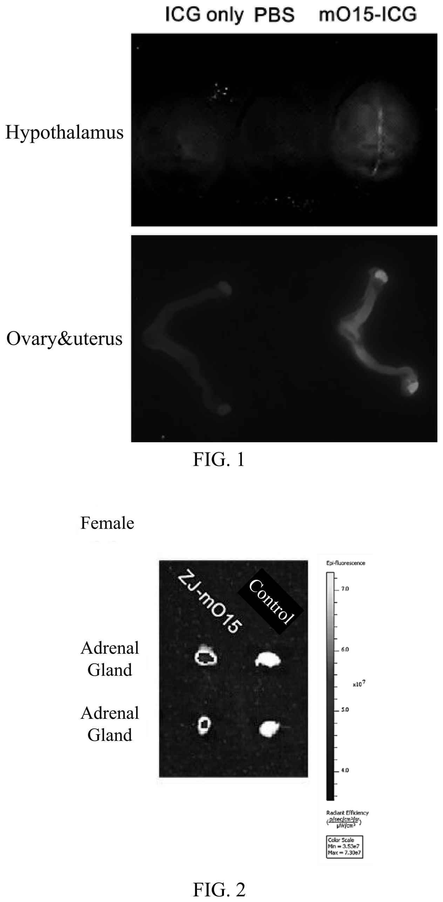

Experimental results are shown in FIGS. 1 and 2. A comparative analysis of the results reveals significant fluorescence signals in brain tissues compared with a control group, indicating that mO15 enters the brains. Significant fluorescence signals are observed in the brain, ovary and uterus, and adrenal gland tissues compared with the control group, indicating that mO15 (SEQ ID NO.2) has specific localization and binding capabilities in hypothalamus, ovary and uterus, and adrenal gland tissues.

### Example 4: Detection of targeting capability of Cy5-labeled polypeptide to brain, gonad, and adipose tissues of male mice

Adult male mice were intravenously injected via tail veins with the following: the Cy5-labeled polypeptide prepared in Example 2, a control polypeptide (a scrambled, irrelevant polypeptide), and a PBS. Each mouse received an injection volume of 100 µL. After 24 hours, detection was performed using a small animal imaging instrument.

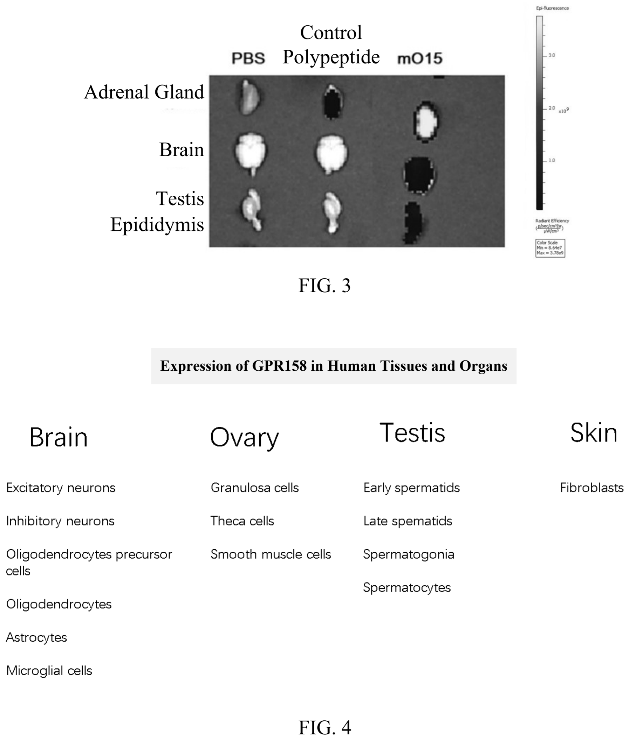

Experimental results are shown in FIG. 3. A comparative analysis of the results from the three groups reveals significant fluorescence signals in brain tissues compared with a control group, indicating that mO15 enters the brains. Significant fluorescence signals are observed in adrenal gland, testis, and epididymis tissues compared with the control group, indicating that the polypeptide has specific localization and binding capabilities in the brain, gonad, and adrenal gland tissues of male mice.

In summary, the results from Examples 3 and 4 demonstrate that the mouse polypeptide is capable of targeting the brain, adrenal gland, and gonad tissues of both female and male mice *in vivo.* Regarding a psychological stress-induced hair loss model and an androgenetic hair loss model, the brains (cerebral cortex neurons) and adrenal glands (corticosteroids) are associated with psychological stress regulation, and the female and male gonads are associated with hormonal regulation. The polypeptide may regulate a hair loss process by acting on target tissues involved in psychological stress regulation and hormonal regulation.

### Example 5: Expression and distribution of polypeptide GPR158 receptor in human brains

The Human Protein Atlas was employed to search for expression and localization of a polypeptide GPR158 receptor in human brains, ovaries, testes, and skins.

Search results are shown in FIG. 4. Single-cell sequencing results from various human tissues indicate that the GPR158 receptor is expressed in the human brains, ovaries, testes, and skins.

### Example 6: Expression and localization of GPR158 receptor in mouse hypothalamus-pituitary-ovary (HPO) axis

Adult female C57 mice were subjected to anatomical dissection to isolate HPO tissues. After section freezing, immunofluorescence staining was performed using a GPR158 antibody.

Experimental results are shown in FIG. 5. It is shown that GPR158 is specifically expressed in subventricular regions of the hypothalami.

Similarly, the results from Examples 5 and 6 demonstrate that the GPR158 receptor is expressed in the brains, female and male gonads, and skins. The polypeptide may regulate a hair loss process by interacting with the GPR158 receptor on these target organs.

### Example 7: Molecular docking simulations of homologous sequence polypeptides from different species with GPR158 receptor

A representative homologous polypeptide sequence was selected from each species, and molecular docking between the representative homologous polypeptide sequences and the GPR158 receptor was simulated by using GalaxyPepDock software.

Simulation results are shown in FIG. 6. The homologous sequence polypeptides from different species have a sequence identity of above 60%, and all the homologous sequence polypeptides bind to an extracellular domain of the human GPR158 receptor (FIG. 6A). All interaction scores are above 20 (FIG. 6B), indicating a strong binding affinity between different polypeptides and the human GPR158 receptor.

The selected polypeptide sequences are as follows:
YLYQWLGAPVPYPDPLEPR SEQ ID No.1 (Ocn19)
YLGASVPSPDPLEPT SEQ ID No.2 (mO15/OC15/mOC15/Ocn15)
LDPGLGAPAPYPDPLEPR SEQ ID No.3 (Livestock species-polypeptide)
NYNRFYNARAAPTPDPLEPL SEQ ID No.4 (Amphibian species-polypeptide)
SNLRNAVFGTPVRDPLESK SEQ ID No.5 (Reptile species-polypeptide)
AGAVTAADLSLTQLESL SEQ ID No.6 (Bony fish species-polypeptide)
YAQDSGVAGAPPNPLEAQ SEQ ID No.7 (Poultry species-polypeptide)
SDFYER YFMHYKTPMEQM SEQ ID No.8 (Bird species-polypeptide)
DHTLVFSNERWPGVS SEQ ID No.9 (Irrelevant polypeptide)

### Example 8: Binding of human osteocalcin Ocn49 (full-length human osteocalcin) and truncated human osteocalcin Ocn19 (SEQ ID NO.1) to GPR158 receptor

The polypeptide prepared in Example 1 was employed in this example, and a binding condition was observed via *in vitro* culture of the polypeptide with a Hela cell line highly expressing a GPR158 receptor.

A specific method involved that Cy5-labeled full-length human osteocalcin Ocn49, truncated human osteocalcin Ocn19 (SEQ ID NO.1), and HeLa cells highly expressing GPR158 were incubated at 4°C, and cells were subsequently washed with PBS. Polypeptides incapable of binding to the cells were considered as non-specific ligands, while polypeptides capable of binding to the cells were considered as specifically binding ligands. An interaction between the polypeptides and the receptor was verified accordingly.

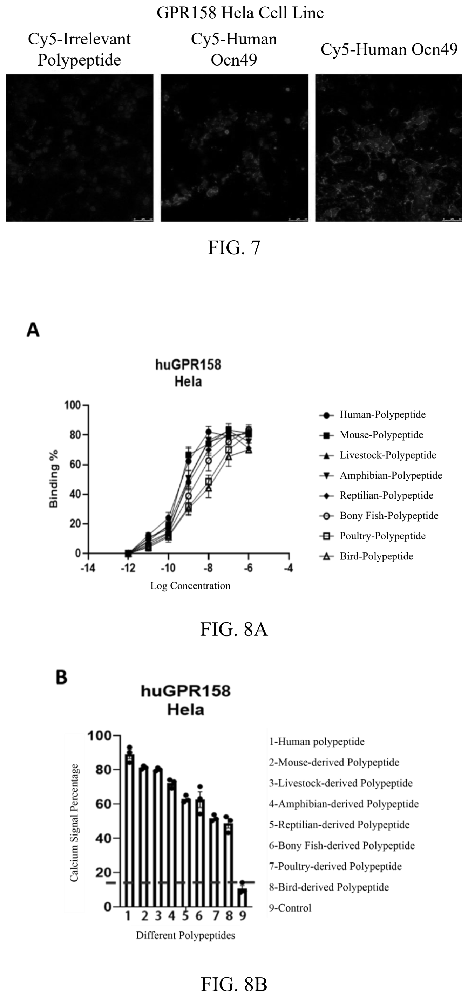

Experimental results are shown in FIG. 7. The results demonstrate that incubation of the polypeptides human Ocn49 and Ocn19 with the GPR158-HeLa cells induces internalization of the GPR158 receptor (red represents the polypeptides located in a cytoplasm). It is indicated that both the polypeptides human Ocn49 and Ocn19 are specifically binding ligands, and that the polypeptides human Ocn49 and human Ocn19 are capable of binding to the GPR158 receptor.

### Example 9: Binding of homologous polypeptides from different species to GPR158 receptor

The polypeptides prepared in Example 7 were employed in this example, and an internalization condition and a calcium signal intensity were observed via *in vitro* culture of the polypeptides with a Hela cell line highly expressing a GPR158 receptor.

Experimental results are shown in FIG. 8. The results show that the homologous polypeptides from different species exhibit a strong binding capacity to human GPR158 receptor cells and are capable of mediating calcium signals generated by the Hela cells highly expressing a GPR158 receptor. It is indicated that although sequences of the osteocalcin-derived peptides vary to a certain extent among species, these variations do not affect binding of the osteocalcin-derived peptides to GPR158 and triggering of downstream signaling pathways. Thus, the polypeptide sequences derived from different species may be used across species.

### Example 10: Psychological stress-induced hair loss mouse model

7-week-old male C57BL/6 mice were treated with application of a corticosteroid Dexamethasone and a glucocorticoid receptor agonist Prednisone (a modeling cycle of 23 days).

Hair at a telogen phase on dorsa was removed, and areas were depilated using a hair removal cream. Mice in one group of the model groups received topical application of a Dexamethasone solution (100 nM, 0.1 mL, dissolved in propylene glycol) to the depilated areas once a day, and mice in the other group received topical application of a Prednisone solution (100 nM, 0.1 mL, dissolved in propylene glycol) to the depilated areas once a day, for 23 consecutive days. Mice in a treatment group were administered with the polypeptide via oral gavage at a concentration of 0.5 mg/kg/day while applied with the drug daily.

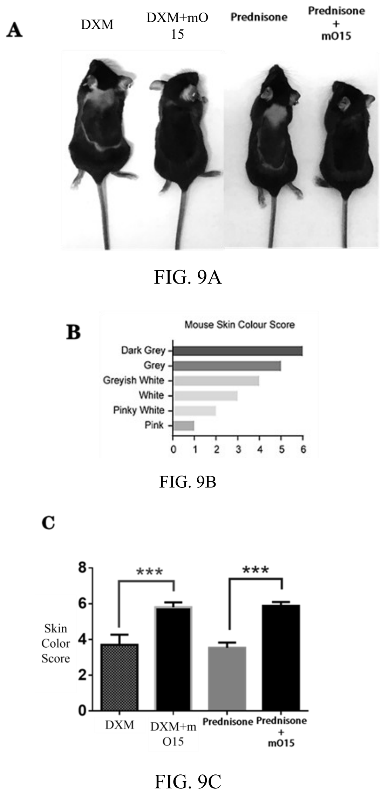

Experimental results are shown in FIG. 9. Comparison of the results from the four groups indicates that the polypeptide is capable of treating hair loss of mice induced by the corticosteroid and the glucocorticoid receptor agonist.

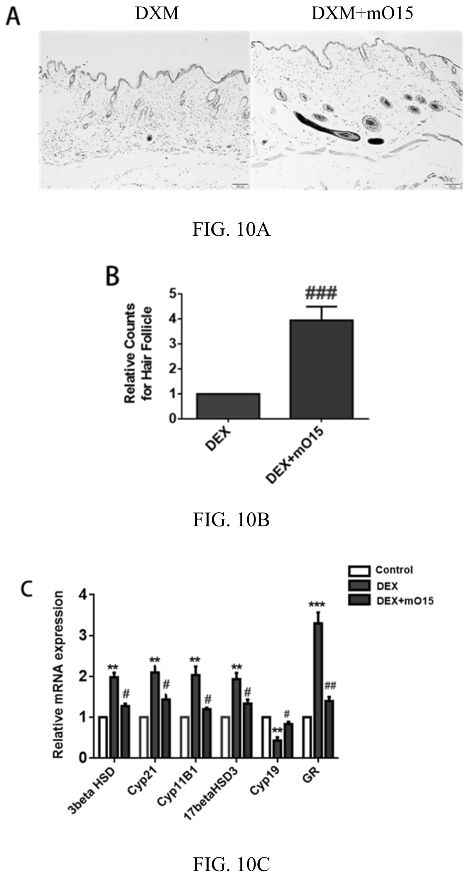

Further, results from section staining and related gene detection of cervicodorsal skins of mice (FIG. 10) show that after treatment with the polypeptide, the number of hair follicles increases significantly; and after treatment with Dexamethasone, expression levels of cortisol synthesis-related enzyme genes 3betaHSD, Cyp21, and Cyp11B1 increase, an expression level of a glucocorticoid receptor gene GR increases significantly, an expression level of a testosterone synthesis-related enzyme gene 17betaHSD3 increases, and an expression level of a estrogen synthesis-related enzyme gene Cyp11 decreases. After administration of mO15, effects of Dexamethasone on the expression levels of the above genes are alleviated.

### Example 11: Androgenetic (seborrheic) hair loss mouse model

7-week-old male C57BL/6 mice were treated with application of DHT (a modeling and experimental cycle of 28 days).

Hair at a telogen phase on 2×2 cm areas of dorsa was removed, and the areas were depilated using a hair removal cream. A DHT solution (0.2%, w/v) was prepared in an ethanol solution (50%, v/v). Mice received topical application of the DHT solution (0.1 mL/cm²) to the depilated areas, once a day, for 28 consecutive days. Mice in the other group received the polypeptide via oral gavage at a concentration of 0.5 mg/kg/day while applied with the DHT daily.

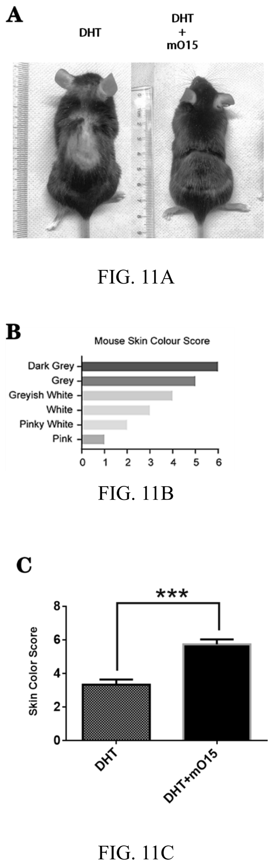

Experimental results are shown in FIG. 11. Comparison of the results from the two groups indicates that the polypeptide is capable of treating hair loss of mice induced by androgens.

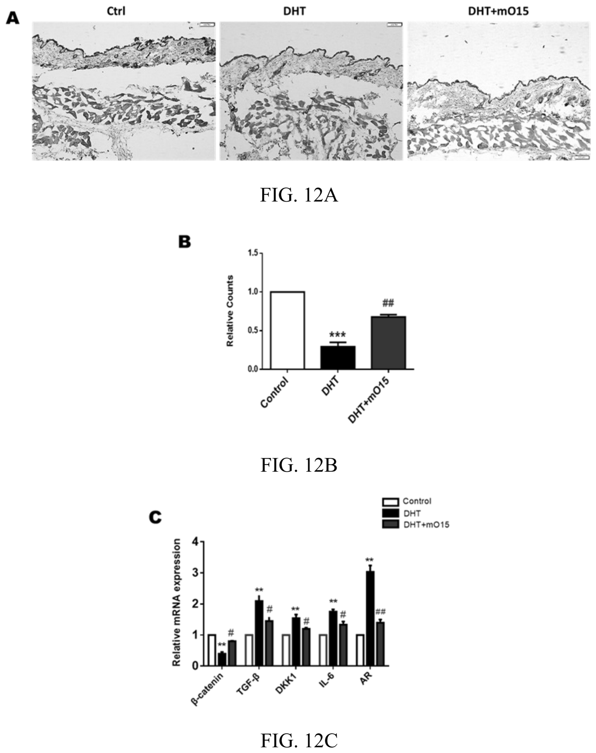

Further, results from section staining and related gene detection of cervicodorsal skins of mice (FIG. 12) show that after treatment with the polypeptide, the number of hair follicles increases significantly; the DHT induces an expression level of a β-catenin gene to decrease and expression levels of TGF-β, DKK1, IL-6, and AR genes to increase; and after treatment with mO15, expression levels of the above genes are alleviated. TGF-β and IL-6 belong to inflammatory factors. Experimental results demonstrate that the androgens lead to inflammatory responses in the skins or hair follicles, and the polypeptide of the present disclosure is capable of significantly reducing the levels of the inflammatory factors, and is suitable for treating seborrheic acne or dermatitis.

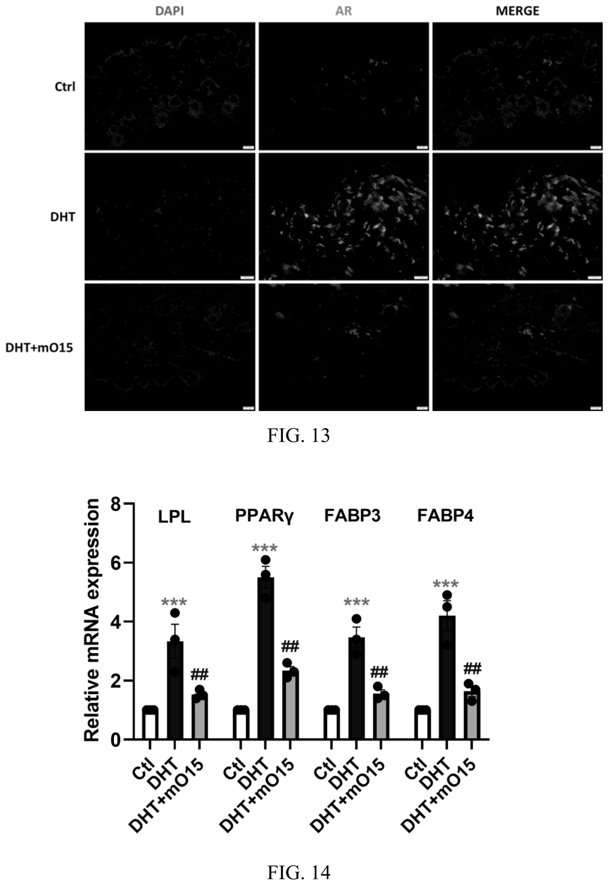

Further, results from immunofluorescence staining of frozen sections of the cervicodorsal skins of mice (FIG. 13) show that an expression level of an AR is significantly increased in the cervicodorsal skins of mice after treatment with the DHT, and after administration of the polypeptide via oral gavage, a DHT-induced high expression level of the AR in the skins is significantly alleviated.

Further, detection results of molecular markers related to sebaceous glands of the cervicodorsal skins of mice (FIG. 14) show that genes related to lipases, adipocyte differentiation, and lipoproteins are significantly increased in the cervicodorsal skins of mice after treatment with the DHT, and after treatment with mO15, expression levels of the above genes are alleviated.

### Example 12: Hair loss phenotype of 10-week-old GPR158KO female mice

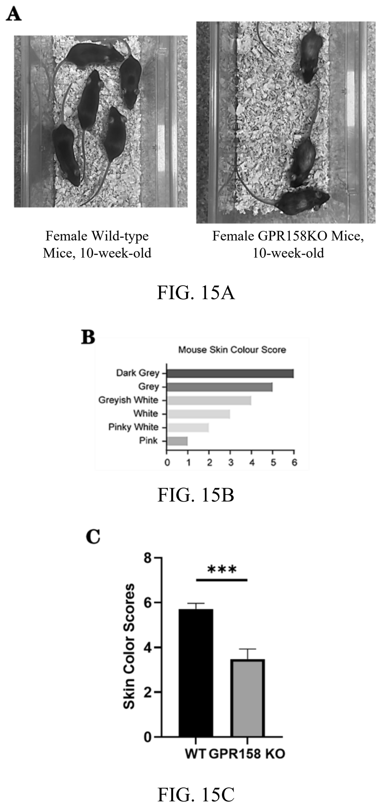

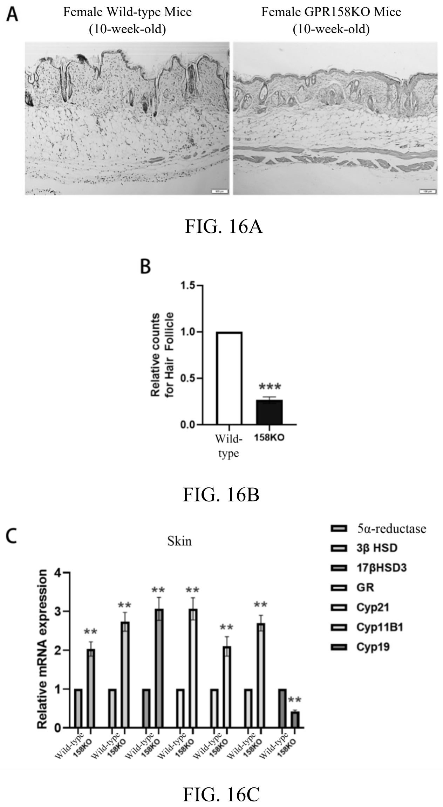

Observations reveal that GPR158KO female mice, i.e., mice with a GPR158 gene knocked out, exhibit a hair loss phenotype at 10 weeks old (FIG. 15). Results from section staining and related gene detection of cervicodorsal skins of wild-type mice and GPR158KO mice at the same age (FIG. 16) show that the number of hair follicles is significantly reduced for the 10-week-old GPR158KO mice; and expression levels of 5α-reductase (which promotes conversion of testosterone to DHT), cortisol synthesis-related enzyme genes 3βHSD, Cyp21, and Cyp11B1, a testosterone synthesis-related enzyme gene 17βHSD3, and a glucocorticoid receptor gene GR are significantly increased, and an expression level of an estrogen synthesis-related enzyme gene Cyp19 is significantly decreased.

The inventors of the present disclosure have discovered for the first time that the GPR158 receptor is associated with hair follicles, and thus a correlation between the GPR158 receptor and the hair loss phenotype is obtained. This correlation may involve regulation of the expression levels of the 5α-reductase, the cortisol synthesis-related enzyme genes 3βHSD, Cyp21, and Cyp11B1, the testosterone synthesis-related enzyme gene 17βHSD3, the glucocorticoid receptor gene GR, and the estrogen synthesis-related enzyme gene Cyp19, thereby influencing the number of hair follicles and hair growth.

### Example 13: Procurement and identification of human primary dermal papilla cells

Human primary dermal papilla cells were purchased from Shanghai Xuanke Biotechnology Co., Ltd. The cells were derived from surgically resected normal scalp tissues and tested positive for fibronectin by immunofluorescence staining (FIG. 17). The cells were identified to have a purity of greater than 90%. The cells were free of HIV-1, HBV, HCV, mycoplasma, bacteria, yeast, and fungi. A fibroblast medium was used as a medium.

### Example 14: Cortisol-induced psychological stress hair loss model

24 hours after serum-starved cells were removed, human primary dermal papilla cells were cultured in a medium containing 1 µM cortisol for 24 hours. Expression levels of cellular molecular markers (including 5α-dehydrogenase, GR, AR, CD200, IL-6, BCL2, and BAX) were detected after 24 hours. Homologous polypeptides from different species (the polypeptides from Example 7) were concurrently added for co-culture, and expression levels of the cellular molecular markers were detected after 24 hours.

Results as shown in FIG. 18 reveal that the expression levels of the glucocorticoid receptor (GR), androgen receptor (AR), and 5α-reductase in the cells are significantly increased after treatment with cortisol; meanwhile, apoptosis of hair follicle cells (indicated by Caspase-3, BCL2, and BAX) is increased, expression levels of a psychological stress-induced phosphoprotein STIP1 and inflammatory factors (TGF-β1 and IL-6) are increased, and an expression level of a hair follicle regression marker MMP-9 is increased; and expression levels of a hair growth marker GAS6 and hair follicle stem cell markers CD200 and CD133 are decreased, and homologous sequence polypeptides from different species are capable of alleviating these changes to a certain extent.

It is indicated that the polypeptides of the present disclosure may be used to alleviate psychological stress-induced hair loss, and particularly hair follicle recession or hair loss caused by psychological stress resulting from apoptosis of hair follicle cells, an aberrantly increased expression level of the phosphoprotein STIP1, aberrantly increased expression levels of the inflammatory factors, and reduced hair follicle growth, as well as acne or dermatitis caused by psychological stress.

### Example 15: DHT-induced androgenetic/seborrheic hair loss cell model

24 hours after serum-starved cells were removed, human primary dermal papilla cells were treated with a high level of androgen by adding 10 nM DHT to a culture medium. Expression levels of cellular molecular markers (including 5α-dehydrogenase, AR, CD200, IL-6, BCL2, BAX, and FABP3) were detected after 24 hours. Homologous polypeptides from different species (the polypeptides from Example 7) were concurrently added for co-culture, and the expression levels of the cellular molecular markers were detected after 24 hours.

Results as shown in FIG. 19 show that expression levels of an androgen receptor (AR), 5α-reductase, androgen synthesis-related enzymes (3βHSD and 17βHSD3) in the cells are significantly increased after treatment with DHT; and meanwhile, apoptosis of hair follicle cells (indicated by Caspase-3, BCL2, and BAX) is increased, expression levels of inflammatory factors (TGF-β, TNF-α, and IL-6) are increased, an expression level of a hair follicle regression marker MMP-9 is increased, an expression level of a hair follicle stem cell marker CD200 is decreased, expression levels of a fatty acid-binding protein FABP3 and an adipogenic differentiation factor PPARγ are increased, and homologous sequence polypeptides from different species are capable of significantly alleviating these changes.

It is indicated that the polypeptides of the present disclosure may be used to alleviate androgenetic hair loss, and particularly androgenetic/seborrheic hair follicle recession or hair loss caused by significantly increased expression levels of the androgen receptor (AR), 5α-reductase, and androgen synthesis-related enzymes (3βHSD and 17βHSD3), apoptosis of hair follicle cells, an aberrantly increased expression level of inflammatory factors, and hair follicle regression, as well as androgenetic/seborrheic acne or dermatitis.

In summary, the present disclosure provides a polypeptide and related substances thereof that may be used for preparing medicaments for treating diseases related to hair follicle recession and/or hair loss caused by psychological stress, and/or androgens, and/or seborrheic factors.

The above descriptions are merely specific embodiments of the present disclosure and are not all embodiments. Any equivalent transformations made to the technical solutions of the present disclosure made by those of ordinary skill in the art through reading the present specification of the present disclosure should be covered by the claims of the present disclosure.

## Claims

1. Use of a polypeptide, polypeptide-derived peptides, or derivatives of the polypeptide and polypeptide-derived peptides for the manufacture of medicament for treating hair follicle recession and/or hair loss caused by psychological stress, seborrheic hair follicle recession and/or hair loss, acne and/or dermatitis caused by psychological stress and seborrheic acne and/or dermatitis;
wherein the polypeptide is selected from an osteocalcin-derived peptide and homologous polypeptides which use the osteocalcin-derived peptide as a skeleton and which are derived from different species, with sequences as follows:
YLYQWLGAPVPYPDPLEPR SEQ ID No.1
YLGASVPSPDPLEPT SEQ ID No.2
LDPGLGAPAPYPDPLEPR SEQ ID No.3
NYNRFYNARAAPTPDPLEPL SEQ ID No.4
SNLRNAVFGTPVRDPLESK SEQ ID No.5
AGAVTAADLSLTQLESL SEQ ID No.6
YAQDSGVAGAPPNPLEAQ SEQ ID No.7
SDFYER YFMHYKTPMEQM SEQ ID No.8
the polypeptide-derived peptides are conserved fragments derived from osteocalcin of different biological species, the polypeptide-derived peptides are located at the same conserved fragment position as any one of the sequences set forth in SEQ ID NO.1-8, and the different biological species are selected from mammal, reptile, amphibian, bony fish, poultry, and bird species; the polypeptide-derived peptides have sequences X₁-X₂-...Xₙ, wherein X represents an amino acid, and n represents any integer from 8 to 25; and the immediately adjacent upstream and downstream sequences of the polypeptide-derived peptides in osteocalcin are both convertase cleavage sites;
the derivatives are products obtained by terminal or side chain modification of the polypeptide or polypeptide-derived peptide, or products obtained by linkage to tags for polypeptide or protein detection or purification, or products obtained by isotope labeling modification;
preferably, the products obtained by modification are selected from products obtained by fluorophore modification, products obtained by phosphorylation modification, products obtained by disulfide bond-based cyclization modification, products obtained by biotin labeling modification, products obtained by photosensitizer modification, products obtained by azide modification, products obtained by polyethylene glycol (PEG) modification, products obtained by methylation modification, products obtained by fluorescence quenching group modification, products obtained by protein conjugation modification, or products obtained by small molecule compound modification; or products obtained by aminylation modification, amidation modification, hydroxylation modification, carboxylation modification, carbonylation modification, alkylation modification, acetylation modification, esterification modification, or glycosylation modification;
preferably, the terminal modification is selected from N-terminal acetylation modification and C-terminal amidation modification of the polypeptide;
preferably, the side chain modification is selected from modification on an R group of an amino acid side chain in the polypeptide;
preferably, fluorescent dye used in the fluorophore modification is selected from aminomethylcoumarin acetic acid (AMCA), fluorescein isothiocyanate (FITC), Rhodamine, Cy3, Cy5, Cy5.5, Cy7, aggregation-induced emission (AIE), and indocyanine green (ICG), and this modification can be used for fluorescence detection;
preferably, the phosphorylation modification is selected from one or more of p-Ser, p-Thr, and p-Tyr;
preferably, the glycosylation modification is introduced at one or more amino acid residues selected from Ser, Asn, Thr, and Tyr;
preferably, the nitrosylation modification is introduced at the Tyr;
preferably, biotin used in the biotin labeling is selected from D-biotin, biotin hydrazide, photobiotin, and biotin-dUTP;
preferably, the photosensitizer modification can be used to prepare photosensitive formulations;
preferably, the azide modification can be used for secondary coupling reactions;
preferably, the PEG modification can be used to prepare drug carriers; and
preferably, an isotope used in the isotope labeling is selected from one or more of ¹³C, ¹⁴C, ¹⁴N, ¹⁵N, ²H, ³H, ¹⁸O, ³²P, ³²S, ³⁴S, ³⁵S, ³⁶S, ³⁵Cl, ³⁷Cl, ¹²⁵I, and ¹³¹I.

2. The use according to claim 1, wherein the hair follicle recession and/or hair loss caused by psychological stress refers to hair follicle recession and/or hair loss of a male or female subject caused by psychological stress;
the seborrheic hair follicle recession and/or hair loss refers to seborrheic hair follicle recession and/or hair loss of a male subject;
the acne and/or dermatitis caused by psychological stress refers to acne and/or dermatitis of a female or male subject caused by psychological stress; and
the seborrheic acne and/or dermatitis refers to seborrheic acne and/or dermatitis of a male subject.

3. Use of substances capable of binding to a GPR158 receptor and modulating its activity for the manufacture of a medicament for treating or preventing hair follicle recession and/or hair loss caused by psychological stress, seborrheic hair follicle recession and/or hair loss, acne and/or dermatitis caused by psychological stress and seborrheic acne and/or dermatitis.

4. The use according to claim 3, wherein the substances capable of binding to the GPR158 receptor and modulating its activity are substances capable of activating downstream signaling pathways of the GPR158 receptor;
preferably, the substances capable of activating downstream signaling pathways of the GPR158 receptor comprise a polypeptide, polypeptide-derived peptides, or derivatives of the polypeptide and polypeptide-derived peptides;
wherein the polypeptide is selected from an osteocalcin-derived peptide and homologous polypeptides which use the osteocalcin-derived peptide as a skeleton and which are derived from different species, with sequences as follows:
YLYQWLGAPVPYPDPLEPR SEQ ID No.1
YLGASVPSPDPLEPT SEQ ID No.2
LDPGLGAPAPYPDPLEPR SEQ ID No.3
NYNRFYNARAAPTPDPLEPL SEQ ID No.4
SNLRNAVFGTPVRDPLESK SEQ ID No.5
AGAVTAADLSLTQLESL SEQ ID No.6
YAQDSGVAGAPPNPLEAQ SEQ ID No.7
SDFYER YFMHYKTPMEQM SEQ ID No.8
the polypeptide-derived peptides are conserved fragments derived from osteocalcin of different biological species, the polypeptide-derived peptides are located at the same conserved fragment position as any one of the sequences set forth in SEQ ID NO.1-8, and the different biological species are selected from mammal, reptile, amphibian, bony fish, poultry, and bird species; the polypeptide-derived peptides have sequences X₁-X₂-...Xₙ, wherein X represents an amino acid, and n represents any integer from 8 to 25; and the immediately adjacent upstream and downstream sequences of the polypeptide-derived peptides in osteocalcin are both convertase cleavage sites;
the derivatives are products obtained by terminal or side chain modification of the polypeptide or polypeptide-derived peptide, or products obtained by linkage to tags for polypeptide or protein detection or purification, or products obtained by isotope labeling modification;
more preferably, the products obtained by modification are selected from products obtained by fluorophore modification, products obtained by phosphorylation modification, products obtained by disulfide bond-based cyclization modification, products obtained by biotin labeling modification, products obtained by photosensitizer modification, products obtained by azide modification, products obtained by polyethylene glycol (PEG) modification, products obtained by methylation modification, products obtained by fluorescence quenching group modification, products obtained by protein conjugation modification, or products obtained by small molecule compound modification; or products obtained by aminylation modification, amidation modification, hydroxylation modification, carboxylation modification, carbonylation modification, alkylation modification, acetylation modification, esterification modification, or glycosylation modification;
more preferably, the terminal modification is selected from N-terminal acetylation modification and C-terminal amidation modification of the polypeptide;
more preferably, the side chain modification is selected from modification on an R group of an amino acid side chain in the polypeptide;
more preferably, fluorescent dye used in the fluorophore modification is selected from AMCA, FITC, Rhodamine, Cy3, Cy5, Cy5.5, Cy7, AIE, and ICG, and this modification can be used for fluorescence detection;
more preferably, the phosphorylation modification is selected from one or more of p-Ser, p-Thr, and p-Tyr;
more preferably, the glycosylation modification is introduced at one or more amino acid residues selected from Ser, Asn, Thr, and Tyr;
more preferably, the nitrosylation modification is introduced at the Tyr;
more preferably, biotin used in the biotin labeling is selected from D-biotin, biotin hydrazide, photobiotin, and biotin-dUTP;
more preferably, the photosensitizer modification can be used to prepared photosensitive formulations;
more preferably, the azide modification can be used for secondary coupling reactions;
more preferably, the PEG modification can be used to prepare drug carriers; and
more preferably, an isotope used in the isotope labeling is selected from one or more of ¹³C, ¹⁴C, ¹⁴N, ¹⁵N, ²H, ³H, ¹⁸O, ³²P, ³²S, ³⁴S, ³⁵S, ³⁶S, ³⁵Cl, ³⁷Cl, ¹²⁵I, and ¹³¹I.

5. A method for preparing an animal model of hair loss or dermatitis, wherein the method comprises knocking out a GPR158 receptor gene in an animal, or treating the animal with substances capable of binding to a GPR158 receptor and inhibiting downstream signaling pathways;
preferably, the substances capable of binding to a GPR158 receptor and inhibiting downstream signaling pathways are selected from inhibitory enzymes, and peptides that bind to the receptor; and antibodies, siRNAs, shRNAs, antisense RNA and DNAzymes, or combinations thereof, and expression vectors comprising siRNAs, shRNAs, and antisense molecules.

6. Use of a substance capable of binding to a GPR158 receptor and modulating its activity for the manufacture of medical cosmetology products, cosmetics, health-care products, foods and additives for preventing and/or treating hair follicle recession, hair loss, acne and dermatitis, of a polypeptide, polypeptide-derived peptides, or derivatives of the polypeptide and polypeptide-derived peptides;
wherein the polypeptide is selected from an osteocalcin-derived peptide and homologous polypeptides which use the osteocalcin-derived peptide as a skeleton and which are derived from different species, with sequences as follows:
YLYQWLGAPVPYPDPLEPR SEQ ID No.1
YLGASVPSPDPLEPT SEQ ID No.2
LDPGLGAPAPYPDPLEPR SEQ ID No.3
NYNRFYNARAAPTPDPLEPL SEQ ID No.4
SNLRNAVFGTPVRDPLESK SEQ ID No.5
AGAVTAADLSLTQLESL SEQ ID No.6
YAQDSGVAGAPPNPLEAQ SEQ ID No.7
SDFYER YFMHYKTPMEQM SEQ ID No.8
the polypeptide-derived peptides are conserved fragments derived from osteocalcin of different biological species, the polypeptide-derived peptides are located at the same conserved fragment position as any one of the sequences set forth in SEQ ID NO.1-8, and the different biological species are selected from mammal, reptile, amphibian, bony fish, poultry, and bird species; the polypeptide-derived peptides have sequences X₁-X₂-...Xₙ, wherein X represents an amino acid, and n represents any integer from 8 to 25; and the immediately adjacent upstream and downstream sequences of the polypeptide-derived peptides in osteocalcin are both convertase cleavage sites;
the derivatives are products obtained by terminal or side chain modification of the polypeptide or polypeptide-derived peptide, or products obtained by linkage to tags for polypeptide or protein detection or purification, or products obtained by isotope labeling modification;
preferably, the products obtained by modification are selected from products obtained by fluorophore modification, products obtained by phosphorylation modification, products obtained by disulfide bond-based cyclization modification, products obtained by biotin labeling modification, products obtained by photosensitizer modification, products obtained by azide modification, products obtained by polyethylene glycol (PEG) modification, products obtained by methylation modification, products obtained by fluorescence quenching group modification, products obtained by protein conjugation modification, or products obtained by small molecule compound modification; or products obtained by aminylation modification, amidation modification, hydroxylation modification, carboxylation modification, carbonylation modification, alkylation modification, acetylation modification, esterification modification, or glycosylation modification;
preferably, the terminal modification is selected from N-terminal acetylation modification and C-terminal amidation modification of the polypeptide;
preferably, the side chain modification is selected from modification on an R group of an amino acid side chain in the polypeptide;
preferably, fluorescent dye used in the fluorophore modification is selected from AMCA, FITC, Rhodamine, Cy3, Cy5, Cy5.5, Cy7, AIE, and ICG, and this modification can be used for fluorescence detection;
preferably, the phosphorylation modification is selected from one or more of p-Ser, p-Thr, and p-Tyr;
preferably, the glycosylation modification is introduced at one or more amino acid residues selected from Ser, Asn, Thr, and Tyr;
preferably, the nitrosylation modification is introduced at the Tyr;
preferably, biotin used in the biotin labeling is selected from D-biotin, biotin hydrazide, photobiotin, and biotin-dUTP;
preferably, the photosensitizer modification can be used to prepare photosensitive formulations;
preferably, the azide modification can be used for secondary coupling reactions;
preferably, the PEG modification can be used to prepare drug carriers;
preferably, an isotope used in the isotope labeling is selected from one or more of ¹³C, ¹⁴C, ¹⁴N, ¹⁵N, ²H, ³H, ¹⁸O, ³²P, ³²S, ³⁴S, ³⁵S, ³⁶S, ³⁵Cl, ³⁷Cl, ¹²⁵I, and ¹³¹I;
preferably, the hair loss comprises psychological stress-induced hair follicle regression and/or hair loss, and androgenetic hair follicle regression and/or hair loss of a female subject and a male subject;
preferably, the prevention and/or treatment of the hair loss refers to promoting hair growth;
preferably, the acne comprises psychological stress-induced acne and androgenetic acne of a female subject and a male subject; and
preferably, the dermatitis comprises psychological stress-induced dermatitis and androgenetic dermatitis of a female subject and a male subject.

7. Use of a substance capable of binding to a GPR158 receptor for the manufacture of detection reagents for targeting brains, adrenal glands, gonadal systems;
preferably, the brains are brains of a female subject and a male subject;
preferably, the adrenal glands are adrenal glands of a female subject and a male subject;
preferably, the male gonadal system comprises a testis and an epididymis;
preferably, the female gonadal system comprises an ovary and a uterus.

8. The use according to claim 7, wherein the substance capable of binding to a GPR158 receptor comprises a polypeptide, polypeptide-derived peptides, or derivatives of the polypeptide and polypeptide-derived peptides;
preferably, the polypeptide is selected from an osteocalcin-derived peptide and homologous polypeptides which use the osteocalcin-derived peptide as a skeleton and which are derived from different species, with sequences as follows:
YLYQWLGAPVPYPDPLEPR SEQ ID No.1
YLGASVPSPDPLEPT SEQ ID No.2
LDPGLGAPAPYPDPLEPR SEQ ID No.3
NYNRFYNARAAPTPDPLEPL SEQ ID No.4
SNLRNAVFGTPVRDPLESK SEQ ID No.5
AGAVTAADLSLTQLESL SEQ ID No.6
YAQDSGVAGAPPNPLEAQ SEQ ID No.7
SDFYER YFMHYKTPMEQM SEQ ID No.8
the polypeptide-derived peptides are conserved fragments derived from osteocalcin of different biological species, the polypeptide-derived peptides are located at the same conserved fragment position as any one of the sequences set forth in SEQ ID NO.1-8, and the different biological species are selected from mammal, reptile, amphibian, bony fish, poultry, and bird species; the polypeptide-derived peptides have sequences X₁-X₂-...Xₙ, wherein X represents an amino acid, and n represents any integer from 8 to 25; and the immediately adjacent upstream and downstream sequences of the polypeptide-derived peptides in osteocalcin are both convertase cleavage sites;
the derivatives of the polypeptide are products obtained by terminal or side chain modification of the polypeptide or polypeptide-derived peptide, or products obtained by linkage to tags for polypeptide or protein detection or purification, or products obtained by isotope labeling modification;
more preferably, the products obtained by modification are selected from products obtained by fluorophore modification, products obtained by phosphorylation modification, products obtained by disulfide bond-based cyclization modification, products obtained by biotin labeling modification, products obtained by photosensitizer modification, products obtained by azide modification, products obtained by polyethylene glycol (PEG) modification, products obtained by methylation modification, products obtained by fluorescence quenching group modification, products obtained by protein conjugation modification, or products obtained by small molecule compound modification; or products obtained by aminylation modification, amidation modification, hydroxylation modification, carboxylation modification, carbonylation modification, alkylation modification, acetylation modification, esterification modification, or glycosylation modification;
more preferably, the terminal modification is selected from N-terminal acetylation modification and C-terminal amidation modification of the polypeptide;
more preferably, the side chain modification is selected from modification on an R group of an amino acid side chain in the polypeptide;
more preferably, fluorescent dye used in the fluorophore modification is selected from AMCA, FITC, Rhodamine, Cy3, Cy5, Cy5.5, Cy7, AIE, and ICG, and this modification can be used for fluorescence detection;
more preferably, the phosphorylation modification is selected from one or more of p-Ser, p-Thr, and p-Tyr;
more preferably, the glycosylation modification is introduced at one or more amino acid residues selected from Ser, Asn, Thr, and Tyr;
more preferably, the nitrosylation modification is introduced at the Tyr;
more preferably, biotin used in the biotin labeling is selected from D-biotin, biotin hydrazide, photobiotin, and biotin-dUTP;
more preferably, the photosensitizer modification can be used to prepare photosensitive formulations;
more preferably, the azide modification can be used for secondary coupling reactions;
more preferably, the PEG modification can be used to prepare drug carriers; and
more preferably, an isotope used in the isotope labeling is selected from one or more of ¹³C, ¹⁴C, ¹⁴N, ¹⁵N, ²H, ³H, ¹⁸O, ³²P, ³²S, ³⁴S, ³⁵S, ³⁶S, ³⁵Cl, ³⁷Cl, ¹²⁵I, and ¹³¹I.

9. Use of a substance capable of binding to a GPR158 receptor and modulating its activity for the manufacture of a medicament, medical cosmetology products, cosmetics, health-care products, foods and additives for inhibiting inflammatory responses caused by elevated corticosteroids or glucocorticoids, or inhibiting inflammatory responses caused by elevated corticosteroids, glucocorticoids, or cortisols.

10. Use of substances capable of binding to a GPR158 receptor and inhibiting functions for the manufacture of products for hair loss-related research models; and
the substances capable of binding to a GPR158 receptor and inhibiting functions are selected from inhibitory enzymes, and peptides that bind to the receptor; and antibodies, siRNAs, shRNAs, antisense RNA and DNAzymes, or combinations thereof, and expression vectors comprising siRNAs, shRNAs, and antisense molecules.

11. A method for treating or preventing hair follicle recession and/or hair loss caused by psychological stress, seborrheic hair follicle recession and/or hair loss, acne and/or dermatitis caused by psychological stress and seborrheic acne and/or dermatitis, wherein the method comprises a step of administering to a subject a polypeptide, polypeptide-derived peptides, or derivatives of the polypeptide and polypeptide-derived peptides, or substances capable of binding to a GPR158 receptor and modulating its activity.
wherein the polypeptide is selected from an osteocalcin-derived peptide and homologous polypeptides which use the osteocalcin-derived peptide as a skeleton and which are derived from different species, with sequences as follows:
YLYQWLGAPVPYPDPLEPR SEQ ID No.1
YLGASVPSPDPLEPT SEQ ID No.2
LDPGLGAPAPYPDPLEPR SEQ ID No.3
NYNRFYNARAAPTPDPLEPL SEQ ID No.4
SNLRNAVFGTPVRDPLESK SEQ ID No.5
AGAVTAADLSLTQLESL SEQ ID No.6
YAQDSGVAGAPPNPLEAQ SEQ ID No.7
SDFYER YFMHYKTPMEQM SEQ ID No.8
the polypeptide-derived peptides are conserved fragments derived from osteocalcin of different biological species, the polypeptide-derived peptides are located at the same conserved fragment position as any one of the sequences set forth in SEQ ID NO.1-8, and the different biological species are selected from mammal, reptile, amphibian, bony fish, poultry, and bird species; the polypeptide-derived peptides have sequences X₁-X₂-...Xₙ, wherein X represents an amino acid, and n represents any integer from 8 to 25; and the immediately adjacent upstream and downstream sequences of the polypeptide-derived peptides in osteocalcin are both convertase cleavage sites;
the derivatives of the polypeptide are products obtained by terminal or side chain modification of the polypeptide or polypeptide-derived peptide, or products obtained by linkage to tags for polypeptide or protein detection or purification, or products obtained by isotope labeling modification;
preferably, the products obtained by modification are selected from products obtained by fluorophore modification, products obtained by phosphorylation modification, products obtained by disulfide bond-based cyclization modification, products obtained by biotin labeling modification, products obtained by photosensitizer modification, products obtained by azide modification, products obtained by polyethylene glycol (PEG) modification, products obtained by methylation modification, products obtained by fluorescence quenching group modification, products obtained by protein conjugation modification, or products obtained by small molecule compound modification; or products obtained by aminylation modification, amidation modification, hydroxylation modification, carboxylation modification, carbonylation modification, alkylation modification, acetylation modification, esterification modification, or glycosylation modification;
preferably, the terminal modification is selected from N-terminal acetylation modification and C-terminal amidation modification of the polypeptide;
preferably, the side chain modification is selected from modification on an R group of an amino acid side chain in the polypeptide;
preferably, fluorescent dye used in the fluorophore modification is selected from AMCA, FITC, Rhodamine, Cy3, Cy5, Cy5.5, Cy7, AIE, and ICG, and this modification can be used for fluorescence detection;
preferably, the phosphorylation modification is selected from one or more of p-Ser, p-Thr, and p-Tyr;
preferably, the glycosylation modification is introduced at one or more amino acid residues selected from Ser, Asn, Thr, and Tyr;
preferably, the nitrosylation modification is introduced at the Tyr;
preferably, biotin used in the biotin labeling is selected from D-biotin, biotin hydrazide, photobiotin, and biotin-dUTP;
preferably, the photosensitizer modification can be used to prepare photosensitive formulations;
preferably, the azide modification can be used for secondary coupling reactions;
preferably, the PEG modification can be used to prepare drug carriers; and
preferably, an isotope used in the isotope labeling is selected from one or more of ¹³C, ¹⁴C, ¹⁴N, ¹⁵N, ²H, ³H, ¹⁸O, ³²P, ³²S, ³⁴S, ³⁵S, ³⁶S, ³⁵Cl, ³⁷Cl, ¹²⁵I, and ¹³¹I.

12. The method according to claim 11, wherein the step of administering a polypeptide or derivatives thereof to a subject comprises administering the polypeptide or derivatives thereof via an oral, intravenous, intramuscular, transdermal, or transmucosal route.

13. A medicament for preventing and/or treating hair follicle regression, hair loss, acne, dermatitis, and related symptoms thereof, wherein the medicament comprises a polypeptide, polypeptide-derived peptides, or derivatives of the polypeptide and polypeptide-derived peptides, and a conjugate that combines a GPR158 receptor agonist, an antagonist, and active ingredients;
the medicament comprises one or more pharmaceutically acceptable carriers;
the carriers are preferably selected from a diluent, an excipient, a filler, a binder, a wetting agent, a disintegrating agent, an absorption enhancer, an adsorbent carrier, a surfactant, and a lubricant;
wherein the polypeptide is selected from an osteocalcin-derived peptide and homologous polypeptides which use the osteocalcin-derived peptide as a skeleton and which are derived from different species, with sequences as follows:
YLYQWLGAPVPYPDPLEPR SEQ ID No.1
YLGASVPSPDPLEPT SEQ ID No.2
LDPGLGAPAPYPDPLEPR SEQ ID No.3
NYNRFYNARAAPTPDPLEPL SEQ ID No.4
SNLRNAVFGTPVRDPLESK SEQ ID No.5
AGAVTAADLSLTQLESL SEQ ID No.6
YAQDSGVAGAPPNPLEAQ SEQ ID No.7
SDFYER YFMHYKTPMEQM SEQ ID No.8
the polypeptide-derived peptides are conserved fragments derived from osteocalcin of different biological species, the polypeptide-derived peptides are located at the same conserved fragment position as any one of the sequences set forth in SEQ ID NO.1-8, and the different biological species are selected from mammal, reptile, amphibian, bony fish, poultry, and bird species; the polypeptide-derived peptides have sequences X₁-X₂-...Xₙ, wherein X represents an amino acid, and n represents any integer from 8 to 25; and the immediately adjacent upstream and downstream sequences of the polypeptide-derived peptides in osteocalcin are both convertase cleavage sites;
the derivatives of the polypeptide are products obtained by terminal or side chain modification of the polypeptide or polypeptide-derived peptides, or products obtained by linkage to tags for polypeptide or protein detection or purification, or products obtained by isotope labeling modification;
preferably, the products obtained by modification are selected from products obtained by fluorophore modification, products obtained by phosphorylation modification, products obtained by disulfide bond-based cyclization modification, products obtained by biotin labeling modification, products obtained by photosensitizer modification, products obtained by azide modification, products obtained by polyethylene glycol (PEG) modification, products obtained by methylation modification, products obtained by fluorescence quenching group modification, products obtained by protein conjugation modification, or products obtained by small molecule compound modification; or products obtained by aminylation modification, amidation modification, hydroxylation modification, carboxylation modification, carbonylation modification, alkylation modification, acetylation modification, esterification modification, or glycosylation modification;
preferably, the terminal modification is selected from N-terminal acetylation modification and C-terminal amidation modification of the polypeptide;
preferably, the side chain modification is selected from modification on an R group of an amino acid side chain in the polypeptide;
preferably, fluorescent dye used in the fluorophore modification is selected from AMCA, FITC, Rhodamine, Cy3, Cy5, Cy5.5, Cy7, AIE, and ICG, and this modification can be used for fluorescence detection;
preferably, the phosphorylation modification is selected from one or more of p-Ser, p-Thr, and p-Tyr;
preferably, the glycosylation modification is introduced at one or more amino acid residues selected from Ser, Asn, Thr, and Tyr;
preferably, the nitrosylation modification is introduced at the Tyr;
preferably, biotin used in the biotin labeling is selected from D-biotin, biotin hydrazide, photobiotin, and biotin-dUTP;
preferably, the photosensitizer modification can be used to prepare photosensitive formulations;
preferably, the azide modification can be used for secondary coupling reactions;
preferably, the PEG modification can be used to prepare drug carriers; and
preferably, an isotope used in the isotope labeling is selected from one or more of ¹³C, ¹⁴C, ¹⁴N, ¹⁵N, ²H, ³H, ¹⁸O, ³²P, ³²S, ³⁴S, ³⁵S, ³⁶S, ³⁵Cl, ³⁷Cl, ¹²⁵I, and ¹³¹I.
